(19) Europäisches Patentamt
European Patent Office
Office européen des brevets

(11) Veröffentlichungsnummer: **0 179 321**
**A2**

(12) **EUROPÄISCHE PATENTANMELDUNG**

(21) Anmeldenummer: **85112481.8**

(22) Anmeldetag: **02.10.85**

(51) Int. Cl.$^4$: **B 01 J 8/06**
//B01J38/02, C07C5/333

(30) Priorität: **25.10.84 DE 3439174**

(43) Veröffentlichungstag der Anmeldung:
**30.04.86 Patentblatt 86/18**

(84) Benannte Vertragsstaaten:
**AT BE DE FR GB IT NL**

(71) Anmelder: **Linde Aktiengesellschaft**
**Abraham-Lincoln-Strasse 21**
**D-6200 Wiesbaden(DE)**

(72) Erfinder: **Hofmann, Karl-Heinz, Dr.-Ing.**
**Tulpenstrasse 71**
**D-8034 Germering(DE)**

(74) Vertreter: **Schaefer, Gerhard, Dr.**
**Linde Aktiengesellschaft Zentrale Patentabteilung**
**D-8023 Höllriegelskreuth(DE)**

(54) **Verfahren und Reaktor zur Durchführung einer endothermen Reaktion.**

(57) Es wird ein Verfahren zur Durchführung einer endothermen Reaktion, beschrieben bei dem ein zu dehydrierender Einsatz durch einen einen Katalysator aufweisenden Reaktor geleitet und der Katalysator nach Desaktivierung regeneriert wird. Um ein derartiges Verfahren mit weniger apparativem und Energieaufwand betreiben zu können, wird vorgeschlagen, daß ein direkt befeuerter Reaktor verwendet und der Einsatz unverdünnt durch diesen Reaktor geleitet wird.

EP 0 179 321 A2

0179321

## Verfahren und Reaktor zur Durchführung einer endothermen Reaktion

Die Erfindung betrifft ein Verfahren und einen Reaktor zur Durchführung einer endothermen Reaktion, bei dem ein zu dehydrierender Einsatz durch einen einen Katalysator aufweisenden Reaktor geleitet und der Katalysator nach Desaktivierung regeneriert wird.

Bei endothermen katalytischen Reaktionen, wie beispielsweise der Dehydrierung von i-Butan, n-Butan oder Gemischen hiervon, wird einem Einsatz Wärme zugeführt, um ihn auf die für die Reaktion benötigte Temperatur zu bringen. Ein derartiges Verfahren ist beispielsweise in Oil and Gas Journal, 28.3.1983, Seiten 75 bis 77 beschrieben. Ein Einsatz, z.B. i-Butan, wird verdampft und vorgewärmt und zusammen mit überhitztem Dampf durch Dehydrierreaktoren geleitet, die in einem gasbefeuerten Brennraum zur Erzeugung der Reaktionstemperatur angeordnet sind. Die Verdünnung des Einsatzes ist nötig, um das Dehydriergleichgewicht zu begünstigen und die erforderliche Dehydriertemperatur zu regulieren. Insbesondere sind dabei mindestens 2 bis 3 Mol Wasserdampf pro Mol Einsatz zur Verdünnung nötig. Darüber hinaus gehende Mengen an

Wasserdampf dienen in erster Linie der Wärmeversorgung. Der verwendete Katalysator ist in Gegenwart von Wasserdampf aktiv. Hierdurch werden einige Vorteile der Wirkung des Dampfes sichtbar. Der Dampfdruck wird z.B. zur Verbesserung der Konvertierungsreaktion gesenkt und es findet eine Reaktion mit Koks statt, wodurch der Katalysator reiner gehalten wird.

Dieses bekannte Verfahren hat jedoch den Nachteil, daß aufgrund der Zugabe der Verdünnungsgasmenge der energetische und apparative Aufwand der Gesamtanlage steigt.

Der Erfindung liegt daher die Aufgabe zugrunde, ein Verfahren der eingangs genannten Art so auszugestalten, daß es mit weniger apparativem und Energieaufwand in wirtschaftlicher Weise betrieben werden kann.

Diese Aufgabe wird erfindungsgemäß dadurch gelöst, daß ein direkt befeuerter Reaktor verwendet und der Einsatz unverdünnt durch diesen Reaktor geleitet wird.

In überraschender Weise hat sich herausgestellt, daß eine Dehydrierung in einem direkt befeuerten Reaktor, beispielsweise Röhrenreaktor mit einstellbaren Wärmestromdichten dann ebenso hohe Ausbeuten bringt, wenn der Einsatz unverdünnt der Reaktion zugeleitet wird, wie im Falle der Einsatzverdünnung.

Die Dehydrierung von beispielsweise i-Butan läuft demnach wie folgt ab: Während einer Zeitspanne von 6 bis 8 Stunden wird unverdünntes i-Butan bei einer Temperatur zwischen 520°C und 620°C, einem Durchsatz GHSV von 150 bis 1200 $Nm^3$ i-Butan/$m^3$ Katalysator, Stunde und einem Druck von 2 - 6 bar katalytisch dehydriert. Dann wird die Heizleistung der Befeuerung zurückgenommen, anschließend beginnt der

Regeneriertakt. Dies ist erforderlich, da sich während des Dehydriervorganges C-Ablagerungen auf dem Katalysator niederschlagen und diesen desaktivieren.

In einem Spültakt werden noch vorhandene Gaskomponenten mittels beispielsweise $N_2$ aus einem Reaktor bzw. der Katalysatormasse entfernt. Sodann wird mit einem $O_2$-haltigen Gas, wie Rauchgas oder Luft, der Katalysator regeneriert, d.h. der Kohlenstoff abgebrannt. Da diese Reaktion exotherm verläuft, muß die freiwerdende Wärme abgeführt werden. Hierzu wird der Katalysator bevorzugt mit einem weiteren Inertgas beaufschlagt, so daß die Wärme konvektiv abgeführt wird. Nach der Regenerierung wird Spülgas zugeführt, um die über der Katalysatormasse befindlichen $O_2$-haltigen Gasmengen zu entfernen. Als Spülgas kann entweder $N_2$ oder ein anderes inertes Gas zur Anwendung kommen.

Im Anschluß an diesen Spültakt wird der Katalysator zur Reaktivierung für eine kurze Zeitspanne mit einem Gas reduzierender Wirkung beaufschlagt. Hierzu kann Einsatz oder ein Teilstrom des Produktes verwendet werden. Nach dieser Reaktivierung ist der Katalysator für einen weiteren Dehydriertakt bereit.

Die geschilderte Verfahrensweise wird mit Vorteil in mindestens drei zyklisch umschaltbaren Katalysatorbetten durchgeführt, wobei in zwei Reaktoren die Dehydrierung abläuft, während einer regeneriert wird.

Der Verzicht auf ein Verdünnungsgas, wie es beispielsweise bei dem weiter oben beschriebenen bekannten Verfahren eingesetzt wird, reduziert den Energiebedarf für das endotherme Verfahren erheblich, da keine Inertgase mitaufgeheizt werden müssen. Damit können in vorteilhafter Weise Wärmeübertragungsflächen eingespart werden. Überdies verringert sich die Produktgasverdichterleistung, weil ebenfalls keine Verdünnungsgase mehr komprimiert werden müssen. Hierdurch vereinfacht sich das Gesamtverfahren.

Bei Durchführung des erfindungsgemäßen Verfahrens erübrigt sich die Abtrennung des Verdünnungsgases vom Produktstrom. Ein weiterer Vorteil besteht darin, daß der apparative Aufwand für Zufuhreinrichtungen für das Verdünnungsgas und Einrichtungen zur Erwärmung desselben nicht mehr erforderlich sind.

Die erfindungsgemäße Verfahrensführung ist anwendbar bei allen endothermen katalytischen Dehydrierprozessen, wie beispielsweise der Dehydrierung von i-Butan, n-Butan und Gemischen davon.

In bevorzugter Weise wird dabei ein deckenbefeuerter Reaktor verwendet, der in seinem Betrieb und seiner Herstellung weniger aufwendig ist. Alternativ besteht auch die Möglichkeit, einen seitenwandbefeuerten oder stufenbefeuerten oder einen eine beliebige Kombination der erwähnten Möglichkeiten aufweisenden Reaktor zu verwenden.

Die Erfindung bezieht sich überdies auf einen Reaktor zur Durchführung des Verfahrens mit einer Einsatzzuleitung und einer Produktleitung sowie in Rohren angeordneter Katalysatormasse.

Dieser Reaktor ist erfindungsgemäß mit einer direkten Befeuerung ausgestattet. Die direkte Befeuerung kann dabei als Deckenbefeuerung, Seitenwandbefeuerung, Stufenbefeuerung oder eine beliebige Kombination der erwähnten Möglichkeiten ausgebildet sein.

Im folgenden sei das erfindungsgemäße Verfahren anhand eines schematisch dargestellten Ausführungsbeispiels näher erläutert.

Über Leitung 1 wird als Einsatz i-Butan mit höchstens 2 Vol.% Verunreinigungen (z.B. $C_3$-$C_4$-Verbindungen) mit einer Temperatur zwischen 520°C und 620°C und einem Druck zwischen 2 und 6 bar herangeführt, mit rückgeführtem Produktgas aus Leitung 2 vermischt, in einem Wärmetauscher 3 gegen Regeneriergas, auf das noch näher eingegangen wird, in einem Wärmetauscher 4 gegen Produktgas und in einem Wärmetauscher 5 indirekt aufgeheizt und einem Reaktor 6, insbesondere einem in dem Reaktor in Rohren 7 angeordneten Katalysator zugeführt.

Der Reaktor 6 ist deckenbefeuert. Hierzu wird über Leitung 8 ein Brennstoff, z.B. ein Brenngas, und über Leitung 30 Luft herangeführt und über Leitungen 9, 10 zur Befeuerung des Reaktors benutzt. Infolge der Beheizung herrscht in dem Katalysatorbett eine Temperatur von 560°C, so daß die endotherme Dehydrierung gemäß

$$C_4H_{10} \rightleftharpoons C_4H_8 + H_2 + \Delta H$$

$$\Delta H = 117 \text{ kJ/mol}$$

bei einem Durchsatz von 950 $Nm^3$ i-Butan/$m^3$ Katalysator, Stunde ablaufen kann. Über Leitung 11 wird sodann Produktgas mit einer Temperatur von 530°C, einem Druck von 2 bar und folgender Zusammensetzung abgezogen:

| | |
|---|---|
| $iC_4H_{10}$ | 28,5 Vol.% |
| $iC_4H_8$ | 28,0 Vol.% |
| $H_2$ | 36,2 Vol.% |
| $CH_4$ | 5,3 Vol.% |
| $C_2H_6$ | 0,3 Vol.% |

$C_3H_8$ 0,9 Vol.%
$C_3H_6$ 0,8 Vol.%

Der Produktstrom wird in den Wärmetauschern 4, 12 und 13 abgekühlt und einer weiteren, nichtdargestellten Auftrennung zugeführt.

Das Rauchgas wird über Leitung 14 aus dem Reaktor 6 abgezogen und mittels eines Gebläses 15 in einen Kamin 16 eingespeist.

Bei der Befeuerung der Reaktorberohrung entsteht Abwärme, die sinnvoll nutzbar gemacht werden kann. Hierzu wird beispielsweise über Leitung 17 Speisewasser herangeführt, im Wärmetauscher 12 bis zum Siedepunkt angewärmt und einer Dampftrommel 18 zugeführt. Dort wird auch der Sattdampf aus Leitung 19 eingespeist. Der Dampf wird über Leitung 20 einem Hochdruckdampfüberhitzer 21 zugeführt und über Leitung 22 als überhitzter Hochdruckdampf abgegeben. Kondensat aus der Dampftrommel 18 wird über Leitung 23 abgezogen, gegen Produkt im Wärmetauscher 12 angewärmt und wieder auf die Dampftrommel aufgegeben.

Parallel zu dem in der Dehydrierphase befindlichen Katalysator ist mindestens ein weiterer Reaktor 24 in Betrieb, der ebenso aufgebaut ist, wie Reaktor 6. Der Anschaulichkeit halber ist jedoch bei Reaktor 24 lediglich eine Berohrung 25 dargestellt, in der die Katalysatormasse angeordnet ist. Über Leitung 26 wird zur Regenerierung des Katalysators ein $O_2$-haltiges Regeneriergas mit einer Temperatur von 450 - 650°C und einem Druck von 2 bis 13 bar herangeführt, mit Stickstoff aus Leitung 27 vermischt, in einem Wärmetauscher 28 gegen verbrauchtes Regeneriergas angewärmt und über den Katalysator geleitet. Dort reagiert der im Regeneriergas enthaltene Sauerstoff unter Energieabgabe mit dem auf dem Katalysator abgelagerten Koks, und wird über Leitung 29 abgezogen, im Wärmetauscher 28 und 3 abgekühlt und zum Kamin gegeben.

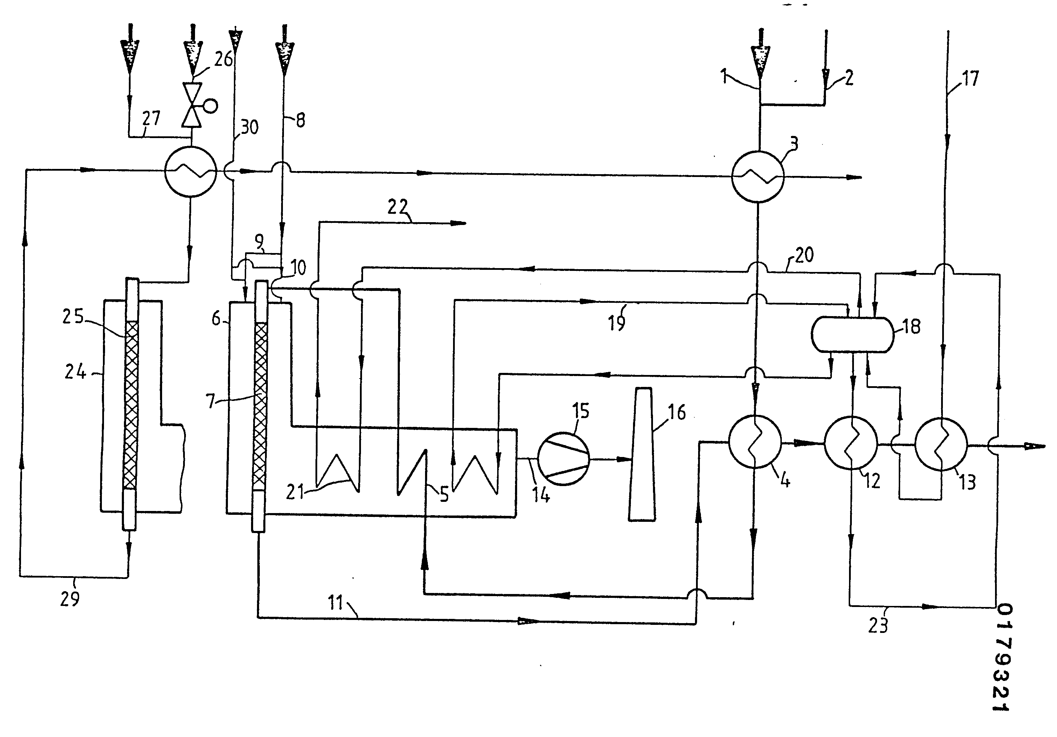

## Patentansprüche

1. Verfahren zur Durchführung einer endothermen Reaktion, bei dem ein zu dehydrierender Einsatz durch einen einen Katalysator aufweisenden Reaktor geleitet und der Katalysator nach Desaktivierung regeneriert wird, dadurch gekennzeichnet, daß ein direkt befeuerter Reaktor verwendet und der Einsatz unverdünnt durch diesen Reaktor geleitet wird.

2. Verfahren nach Anspruch 1, dadurch gekennzeichnet, daß ein deckenbefeuerter Reaktor verwendet wird.

3. Verfahren nach Anspruch 1, dadurch gekennzeichnet, daß ein seitenwandbefeuerter Reaktor verwendet wird.

4. Verfahren nach Anspruch 1, dadurch gekennzeichnet, daß ein stufenbefeuerter Reaktor verwendet wird.

5. Reaktor zur Durchführung des Verfahrens nach Anspruch 1 mit einer Einsatzzuleitung und einer Produktleitung sowie in Rohren angeordneter Katalysatormasse, dadurch gekennzeichnet, daß der Reaktor mit einer direkten

Befeuerung ausgestattet ist.

6. Reaktor nach Anspruch 5, dadurch gekennzeichnet, daß die Befeuerung als Deckenbefeuerung ausgebildet ist.

7. Reaktor nach Anspruch 5, dadurch gekennzeichnet, daß die Befeuerung als Seitenwandbefeuerung ausgebildet ist.

8. Reaktor nach Anspruch 5, dadurch gekennzeichnet, daß die Befeuerung als Stufenbefeuerung ausgebildet ist.

0179321